Europäisches Patentamt

European Patent Office

Office européen des brevets

(19)

(11) Veröffentlichungsnummer: **0 208 841 B1**

(12) # EUROPÄISCHE PATENTSCHRIFT

(45) Veröffentlichungstag der Patentschrift: 18.09.91

(51) Int. Cl.5: **A61M 25/00**

(21) Anmeldenummer: **86104909.6**

(22) Anmeldetag: **10.04.86**

(54) Harnleiterkatheter.

(30) Priorität: **13.07.85 DE 3525165**

(43) Veröffentlichungstag der Anmeldung:
**21.01.87 Patentblatt 87/04**

(45) Bekanntmachung des Hinweises auf die
Patenterteilung:
**18.09.91 Patentblatt 91/38**

(84) Benannte Vertragsstaaten:
**AT BE CH DE FR GB IT LI LU NL SE**

(56) Entgegenhaltungen:
**DE-U- 7 904 093
US-A- 4 227 533
US-A- 4 240 434
US-A- 4 571 241**

(73) Patentinhaber: **B. Braun-SSC AG
Gerliswilstrasse 74
CH-6020 Emmenbrücke(CH)**

(72) Erfinder: **Gerlach, Roland
Kirchweg 9
W-3501 Guxhagen(DE)**

(74) Vertreter: **von Kreisler, Alek, Dipl.-Chem. et al
Deichmannhaus am Hauptbahnhof
W-5000 Köln 1(DE)**

## Beschreibung

Die Erfindung betrifft einen Harnleiterkatheter aus einem Schlauch, der am nierenseitigen Ende und am blasenseitigen Ende mindestens eine Öffnung aufweist.

Es ist bekannt zur Schienung des Harnleiters und zur inneren Urinableitung von der Niere zur Blase Harnleiterkatheter zu verwenden (DE-GM 79 04 093). Solche Harnleiterkather bestehen aus einem Schlauch, der entweder durch Einrollen der beiden Katheterenden verformt ist oder kleine schräg angeklebte Laschen als Widerhaken und eine Fixierplatte am blasenseitigen Ureterrostium aufweist. Die Katheter weisen am nierenseitigen Ende und teilweise auch im mittleren Teil Drainagebohrungen zur Ableitung des Urins in das Katheterlumen und von dort in die Blase auf. Ebenso ist das blasenseitige Ende mit kleinen Bohrungen versehen.

Während der Füllungsphase der Blase strömt der Urin antegrad von der Niere zur Blase. Bei einer Erhöhung des Detrusordruckes, z.B. bei Miktion, über ca. 20 cm Wassersäule tritt ein vesicorenaler Reflux auf. Dabei strömt der Urin durch das Katheterlumen retrograd in den Nierenhohlraum. Ein Reflux im Ringspalt zwischen Katheter und Urether konnte nicht beobachtet werden. Der Blaseninnendruck kann bei der Miktion über einen Zeitraum von mehreren Minuten Werte von über 150 cm Wassersäule annehmen. Bei neurogen gestörten Harnblasen, z.B. Urge-Blasen, können diese Werte bei hohen Kontraktionsfrequenzen (bis zu 4/min) auftreten. In diesen Fällen tritt ein Reflux in das Nierenbecken auf, der durch die hohen Drücke eine starke Ballonierung hervorruft, die neben den ascendierenden Bakterien eine hochgradige Gefährdung des Patienten darstellen können.

Bei infiziertem Blasenurin werden die Keime in das Nierenbecken gespült, so daß es zu einer Infektion kommen kann. Die Druckerhöhung im Nierenbecken durch den retrograd strömenden Urin kann starke Flankenschmerzen hervorrufen.

Aus FR-A-2 133 083 ist eine Harnleiterprothese bekannt, die für einen totalen Harnleiterersatz bestimmt ist. Diese Prothese besteht aus einem relativ dickwandigen Rohr aus Elastomermaterial, welches an den Endabschnitten mit Textilmanschetten zum Zurückhalten in der Niere bzw. der Blase versehen ist. Am blasenseitigen Ende befindet sich ein Entenschnabelventil zur Verhinderung des Urinrückflusses, welches aus einem dünnwandigen Rohr besteht, dessen freies Ende an einer oder mehreren axialen Flächen abgeplattet ist. Ein solches Entenschnabelventil hat den Nachteil, daß es einerseits nicht vollständig schließt und daß es andererseits zum völligen Öffnen einen nicht unerheblichen Druck erfordert.

US-A-4 240 434 beschreibt eine peritoneo-venöse Umgehung für die Behandlung von Bauchwassersucht. Diese Umgehung führt vom Peritoneum zu einer Vene und sie enthält eine subkutan zu verlegende Ballonpumpe. Der vom Peritoneum in die Ballonpumpe hinführende Katheterteil ist innerhalb der Ballonpumpe mit einem Rückschlagventil versehen, welches als Folienventil aus zwei dünnwandigen, an ihren seitlichen Rändern miteinander verklebten oder verschweißten Folien ausgebildet und an dem Schlauch angebracht ist. Dieses Rückschlagventil bildet das Ansaugventil der Ballonpumpe und es verhindert, daß angesaugte Aszites-Flüssigkeit beim Zusammendrücken der Pumpe wieder in das Peritoneum zurückgedrückt wird.

Der Erfindung liegt die Aufgabe zugrunde, einen Harnleiterkatheter der eingangs genannten Art derart auszubilden, daß die Infektionsgefahr des Nierenraums verringert wird.

Die Lösung dieser Aufgabe erfolgt erfindungsgemäß mit den Merkmalen des Patentanspruchs 1.

Durch die Anbringung des Rückschlagventils am blasenseitigen Katheterende wird ein Urinfluß nur in antegrader Strömungsrichtung zugelassen und der vesicorenale Rückfluß wird verhindert. Bei dem erfindungsgemäßen Harnleiterkatheter wird auf die sonst üblichen zahlreichen Bohrungen am blasenseitigen Katheterende verzichtet. Diese Bohrungen bieten strömungsdynamisch für die Drainage keinen Vorteil, sondern sie sind sogar nachteilig, weil sie aufgrund ihrer Kanten Kristallisationskeime für eine mögliche Inkrustation des Katheters bilden.

Da eine Erhöhung des für die Katheterströmung verantwortlichen Nierenbeckendruckes unter allen Umständen zu vermeiden ist, muß der Öffnungsdruck des Rückschlagventils sehr niedrig sein, nämlich kleiner als 1 cm Wassersäule. Weiterhin ist die Baugröße des Ventils begrenzt, da der Katheter endoskopisch gelegt wird. Um diese Anforderungen zu erfüllen, ist das Rückschlagventil vorzugsweise ein Folienventil. Das Lumen des Folienventils sollte bei maximaler Öffnung mindestens gleich dem Lumen des Schlauchs sein.

Das Folienventil besteht aus zwei dünnwandigen, an ihren Rändern miteinander verklebten oder verschweißten Folien.

Inkrustationen der inneren Splints treten im wesentlichen in Abhängigkeit von der Zusammensetzung des Patientenurins auf. Untersuchungen an zahlreichen ausgewählten Materialien haben ergeben, daß Silikon und Polyurethan die geringste Inkrustationstendenz aufweisen. Dabei bietet Polyurethan aufgrund seiner höheren Festigkeit gegenüber Silikon den Vorteil, daß bei gleichem Außendurchmesser das innere Lumen deutlich größer gewählt werden kann.

Das sehr dünne Folienpaar des Folienventils wird bei jeder Urinejakulation in die Blase geöffnet und verschließt sich anschließend wieder. Die stetigen Wandbewegungen der dünnwandigen Folien bewirken, daß großflächige Ablagerungen abbrechen und auf natürlichem Wege abgespült werden.

Im folgenden wird ein Ausführungsbeispiel der Erfindung unter Bezugnahme auf die Zeichnungen näher erläutert.

Es zeigen:

Fig. 1    eine schematische Darstellung eines im Harnleiter verlegten Harnleiterkatheters und

Fig. 2    ein Schnitt entlang der Linie II-II von Fig. 1.

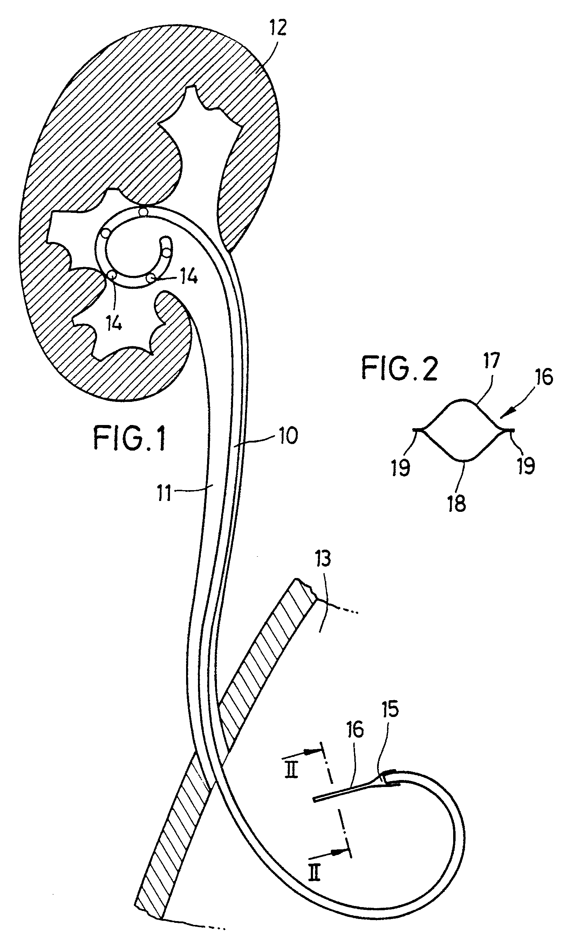

Der dargestellte Harnleiterkatheter besteht aus einem flexiblen Schlauch 10, der zur inneren Schienung des Harnleiters 11 in diesen eingeführt wird. Das distale Ende des Schlauches 10 befindet sich in der Niere 11 und das proximale Ende in der Blase 13. Jedes der beiden Enden ist in bekannter Weise nach Art eines Schweineschwanzes spiralförmig gebogen, so daß es sich in dem betreffenden Organ verankert, um eine Sicherung gegen Wandern des Harnleiterkatheters zu bewirken. Am nierenseitigen Ende des Schlauchs 10 sind mehrere Drainageöffnungen 14 in der Schlauchwand vorgesehen. Am blasenseitigen Ende sind keine seitlichen Öffnungen vorgesehen, sondern es ist nur eine einzige Öffnung 15 am Schlauchende vorhanden. An diesem Schlauchende ist das Folienventil 16 angebracht, das die Öffnung 15 umgibt. Das Folienventil 16 besteht aus zwei sehr dünnwadigen Folienteilen 17, 18, die an ihren seitlichen Rändern 19 miteinander verklebt oder verschweißt sind. Das eine Ende des Folienventils 16 umgibt das Ende des Schlauchs 10 abdichtend. Die Folienteile sind hier mit dem Schlauch verklebt oder verschweißt. Die im Schließzustand flach gegeneinanderliegenden Folienteile 17 und 18 sind an dem freien Ende des Folienventils nicht miteinander verbunden, so daß sie sich bei einem Innendruck im Schlauch 10 gemäß Fig. 2 wie die Lippen eines Mundes öffnen können. Wegen der Weichheit der Folien öffnet das Folienventil schon bei einem Überdruck von weniger als 1 cm Wassersäule den Schlauch 10. Der Urin kann daher ungehindert von der Niere 12 zur Blase 13 fließen.

Das Einführen des Katheters in den Körper erfolgt vorzugsweise durch eine Einführröhre hindurch. Es ist aber auch möglich, den Katheter nach der Seldinger-Technik über einen Führungsdraht zu schieben, der anschließend wieder herausgezogen wird. Von der jeweiligen Einführtechnik hängt es ab, ob das nierenseitige Katheterende offen oder geschlossen ausgebildet ist.

Das Folienventil 16 kann auf dem Katheterende auch reibschlüssig festgehalten werden, wenn es mit entsprechender Spannung auf das Katheterende aufgezogen wird.

## Patentansprüche

1. Harnleiterkatheter bestehend aus einem Schlauch (10), der am nierenseitigen Ende und am blasenseitigen Ende mindestens eine Öffnung (14,15) aufweist, **dadurch gekennzeichnet,** daß die einzige Öffnung (15) am blasenseitigen Ende durch ein nur nach außen hin durchlässiges Rückschlagventil verschlossen ist, welches als Folienventil (16) aus zwei dünnwandigen, an ihren seitlichen Rändern miteinander verklebten oder verschweißten Folien (17,18) ausgebildet und an dem Schlauch (10) angebracht ist.

2. Harnleiterkatheter nach Anspruch 1, dadurch gekennzeichnet, daß das Lumen des Folienventils (16) bei maximaler Öffnung mindestens gleich dem Lumen des Schlauchs (10) ist.

3. Harnleiterkatheter nach Anspruch 1 oder 2, dadurch gekennzeichnet, daß das Folienventil (16) aus Silikon oder Polyurethan besteht.

## Claims

1. A urethral catheter comprising a tube (10) having its kidney-side end and its bladder-side end provided with at least one opening (14,15), **characterized in** that the sole opening (15) at the bladder-side end is closed only by a back-check valve opening only to the outside and being provided as a web valve (16) consisting of two thinwalled foil web sheets (17,18) having their lateral edges bonded or welded to each other, and that the back-check valve is attached to the tube (10).

2. The urethral catheter according to claim 1, characterized in that, in the fully opened state of the web valve (16), the lumen of the web valve (16) is at least equal to the lumen of the tube (10).

3. The urethral catheter according to claim 1 or 2, characterized in that the web valve (16) is made of silicone or polyurethane.

## Revendications

1. Cathéter urétéral constitué par un tuyau souple (10), qui possède au moins une ouverture (14,15) à l'extrémité côté rein et à l'extrémité côté vessie, caractérisé en ce que l'ouverture unique (15) située sur l'extrémité côté vessie est fermée par une soupape antiretour, qui permet une circulation uniquement en direction de l'extérieur, est constituée sous la forme d'une soupape à feuilles (16) constituée de deux feuilles minces (17,18) collées ou soudées l'une à l'autre au niveau de leurs bords latéraux, et est fixée au tuyau souple (10).

2. Cathéter urétéral selon la revendication 1, caractérisé en ce que l'ouverture de passage dans la soupape à feuilles (16) lors de l'ouverture maximale de cette dernière est au moins égale à l'ouverture de passage dans le tuyau souple (10).

3. Cathéter urétéral selon la revendication 1 ou 2, caractérisé en ce que la soupape à feuilles (16) est réalisée en silicone ou en polyuréthane.

EP 0 208 841 B1

FIG.1

FIG.2